# EUROPEAN PATENT APPLICATION

(11) **EP 4 310 773 A1**
(43) Date of publication of application: **24.01.2024**
(21) Application number: 22185419.3
(22) Date of filing: 18.07.2022
(51) Int. Cl.: G06T 7/00, G06T 7/12, G06T 7/149, G06T 7/62, G16H 30/40

(54) **ULTRASOUND DATA PROCESSING**

(71) Applicant: Koninklijke Philips N.V., 5656 AG Eindhoven (NL)
(72) Inventor: WAECHTER-STEHLE, Irina, Eindhoven (NL); GESSERT, Nils Thorben, Eindhoven (NL); WEHLE, Simon, 5656AG Eindhoven (NL); PETERS, Jochen, 5656AG Eindhoven (NL)
(74) Representative: Philips Intellectual Property & Standards

(57) **Abstract**

A method and device for maintaining and updating a shared register storing one or more parameters related to features of an anatomical structure, accessible by multiple different software applications adapted for deriving quantification data from ultrasound images using the one or more parameters, such that updates to the parameters can be shared by all of the applications, avoiding a need for a same parameter to be re-computed multiple different times, and avoiding a need for a user to reenter an updated parameter into each separate application.

## Description

### FIELD OF THE INVENTION

This method relates to the field of ultrasound data processing.

### BACKGROUND OF THE INVENTION

It is known to apply dedicated image processing software modules to ultrasound imaging data which are configured to obtain specific outputs based on the image data. For example, there exist modules for obtaining specific anatomical measurements from a set of ultrasound image data, e.g. dimensions associated with heart, or standard dimensions associated with a fetus, or dynamic measurements associated with an organ such as the heart, e.g. cardiac output. There are applications which process the data to synthesize new image views or to modulate the imagery to better represent certain features, e.g. segmentation, or artificial contrast enhancement.

### SUMMARY OF THE INVENTION

In real-world clinical use, multiple such software applications may typically be applied to a given dataset to obtain all required information. For example, in relation to a single organ, such as the heart, there may be a multiplicity of different applications which quantify different information associated with the heart, or generate specific imagery associated with the heart.

It is the recognition of the inventors that, within this context, there is often duplication of computation steps between the multiple applications, since multiple applications might make use of a same one or more parameters associated with the anatomical structure of interest. It has been the realization of the inventors that there is an opportunity to improve efficiency therefore through providing a data sharing solution through which the computation and subsequent updating of common parameters utilized by multiple software modules can be collectively pooled, to avoid duplication of processing. There is also an opportunity to improve reliability of outputs of the software modules, since in some cases algorithms or processing steps can fail or produce bad results if they do not have access to certain anatomical information.

The invention is defined by the claims.

According to examples in accordance with an aspect of the invention, there is provided a computer-implemented method comprising: receiving ultrasound imaging data of an anatomical structure of a patient obtained from an ultrasound acquisition system; communicating with an anatomical feature register which stores one or more parameters associated with an anatomical feature of the anatomical structure; a plurality of application modules each processing the obtained ultrasound imaging data based on a current value of at least one of the one or more parameters associated with an anatomical feature currently stored in the anatomical feature register to produce output quantification or segmentation data; and receiving input data from a user interface.

The method further comprises: dynamically updating the value of at least one of the one or more parameters stored in the anatomical feature register based on the input data received from the user interface and/or the output quantification or segmentation data produced by any of the plurality of application modules.

The invention thus proposes a system (and related method and computer program) for enabling a plurality of application modules to process ultrasound imaging data of a structure of a patient in an efficient manner, so that any update or correction made to a parameter associated with a feature in the context of a given application module can be readily used by the other application modules. This avoids a need for parameters to be recomputed multiple times, and avoids a need for a user to re-enter an updated parameter multiple times into each separate application. In the latter case, the user can perform one data input action for updating or setting a parameter value, and this automatically updates or sets the parameters for all of the application modules. Furthermore, in some cases, it could be that certain algorithms or processing steps can fail or produce bad results if they do not have access to certain anatomical information. For example, a cardiac segmentation model such as the Philips HeartModel may need a heart size as an input in order to correctly segment very small or large hearts. Thus, prevention of algorithm failure or bad algorithm results is a further advantage according to some embodiments.

The term 'application module' means for example a software module or hardware module encoded with software, in either case being operable to perform processing of the ultrasound image data to produce output quantification or segmentation data. It may comprise or take the form of one or more algorithms for performing the method steps ascribed to it. It may comprise or take the form of a program or routine for performing the one or more method steps ascribed to it.

Output quantification data may, by way of non-limiting example, include quantifications of one or more anatomical measurements such as dimensions or size parameters of the anatomical structure. These may include length parameters, area parameters, or volume parameters for example. Output quantification data may include dynamic measurements, e.g. hemodynamic measurements in the case of cardiac imaging, such as cardiac output.

In some embodiments, the method further comprises generating initialization data for at least one of the one or more parameters of the anatomical feature, and storing the initialization data in the anatomical feature register as an initial value for the at least one parameter. In some embodiments, the initialization data is generated based on the received ultrasound imaging data. In some embodiments, the initialization data may be generated based on retrieving the initialization data from a datastore. In some embodiments, the initialization data may be generated based on applying at least one of the plurality of application modules to the received ultrasound imaging data, or based on applying at least one of the plurality of application modules to historical ultrasound imaging data for the patient retrieved from a datastore.

In some embodiments, the method further comprises receiving an exam start indicator signal indicative of a start of an ultrasound examination session, for example from the ultrasound acquisition system. In some embodiments, the generating and storing the initialization data is performed responsive to receipt of the exam start indicator signal. In this way, at the start of the examination of the patient, an initial value of the at least one parameter is obtained. This initial value may be updated as the examination proceeds.

In some embodiment, the generating the initialization data for the at least one of the one or more parameters comprises applying an artificial intelligence algorithm to the received ultrasound imaging data. In some embodiments, the artificial intelligence algorithm is a convolutional neural network.

In some embodiments, for at least a subset of the plurality of application modules, the output quantification or segmentation data may include a segmentation of at least one anatomical feature or structure. This may be in the form of a segmentation mask or mesh for the feature or structure. This might be stored in the register as a series of co-ordinate points corresponding to the mesh or mask vertex points.

In some embodiments, at least a subset of the application modules are each adapted to generate a segmentation based on an initial shape template, and wherein the initial shape template is scaled according to a scaling parameter, and wherein the scaling parameter is, or is computed based on, at least one of the one or more parameters stored in the anatomical feature register.

In some embodiments, the anatomical structure is a cardiac structure.

In some embodiments, the one or more parameters include one or more of: a heart size, a left ventricle size, a left atrium size, a right ventricle size, a right atrium size at end diastole, ejection faction and/or a left ventricle length.

In some embodiments, the received ultrasound imaging data comprises 2D ultrasound imaging data for one or a series of time frames. In some embodiments, the received ultrasound imaging data comprises 3D ultrasound imaging data for one or a series of time frames.

In some embodiments, the anatomical feature register includes an update counter entry associated with at least one of the one or more parameters, and wherein concurrently with any updating of the parameter value in the anatomical feature register based on the user input data, the update counter entry is incremented. In some embodiments, any update of the parameter in the anatomical feature register is blocked responsive to a current value of the update counter register exceeding a threshold. In some embodiments, this threshold is zero. In this way, once the parameter has been corrected or updated once, other applications are blocked from further updating it. This avoids duplication of processing, and also ensures consistency in the use of the same parameter.

In some embodiments, a reset indicator signal is generated at an end or at a beginning of any ultrasound examination session, and wherein the method comprises resetting the update counter entry to zero responsive to the reset indicator signal.

The invention can also be embodied in software. Thus, another aspect of the invention is a computer program product comprising code means configured, when executed by a processor, to perform a method in accordance with any embodiment or example described in this document, or in accordance with any claim of this application.

The invention can also be embodied in hardware form. Thus, another aspect of the invention is a processing unit, comprising: an input/output for connection in use with an ultrasound data acquisition apparatus, for receiving ultrasound imaging data of an anatomical structure of a patient; an anatomical feature register adapted to store one or more parameters associated with an anatomical feature of the anatomical structure; a control module operatively coupled to the anatomical feature register; and a plurality of application modules operatively coupled to the control module, each application module being adapted to process ultrasound imaging data received at the input/output based on a current value of at least one of the one or more parameters stored in the anatomical feature register to produce output quantification or segmentation data. The aforementioned control module is adapted to: receive user input data from a user interface; and dynamically update the value of at least one of the one or more parameters stored in the anatomical feature register based on the user input data provided by the user through the user interface and/or the output quantification or segmentation data produced by any of the plurality of application modules.

Any of the features or options described in relation to the method aspect of the invention can be applied equally to the software and hardware aspects of the invention.

In some embodiments, the processing unit further comprises a register initialization module adapted to generate initialization data for at least one of the one or more parameters based on the received ultrasound imaging data, the initialization data comprising an initial value for at least one of the one or more parameters.

In some embodiments, the control module is further adapted to trigger the initialization module to generate the initialization data and store the data as an initial value for at least one of the one or more parameters in the anatomical feature register.

In some embodiments, the anatomical structure is a cardiac structure. In some embodiments, the one or more parameters include one or more of: a heart size, a left ventricle size, a left atrium size, a right ventricle size, a right atrium size at end diastole, ejection faction and/or a left ventricle length.

Another aspect of the invention is a system comprising: a processing unit in accordance with any embodiment described in this document, e.g. as outlined above; and an ultrasound data acquisition apparatus operably coupled with the input/output of the processing unit. In some embodiments, the system further comprises a user interface for obtaining a user input.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 outlines steps of an example method in accordance with one or more embodiments of the invention;
Fig. 2 schematically outlines components and a processing flow of an example processing arrangement in accordance with one or more embodiments of the invention; and
Fig. 3 illustrates results of a convolutional neural network used to generate initialization data.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method and device for maintaining and updating a shared register storing one or more parameters related to features of an anatomical structure, accessible by multiple different software applications adapted for deriving quantification data from ultrasound images using the one or more parameters, such that updates to the parameters can be shared by all of the applications, avoiding a need for a same parameter to be re-computed multiple different times, and avoiding a need for a user to re-enter an updated parameter into each separate application.

Thus, an overall aim of embodiments of the present invention is to provide improved connection between different applications. It is an aim to ensure that information can more easily flow from one application to the next, in particular if corrections are made by the user.

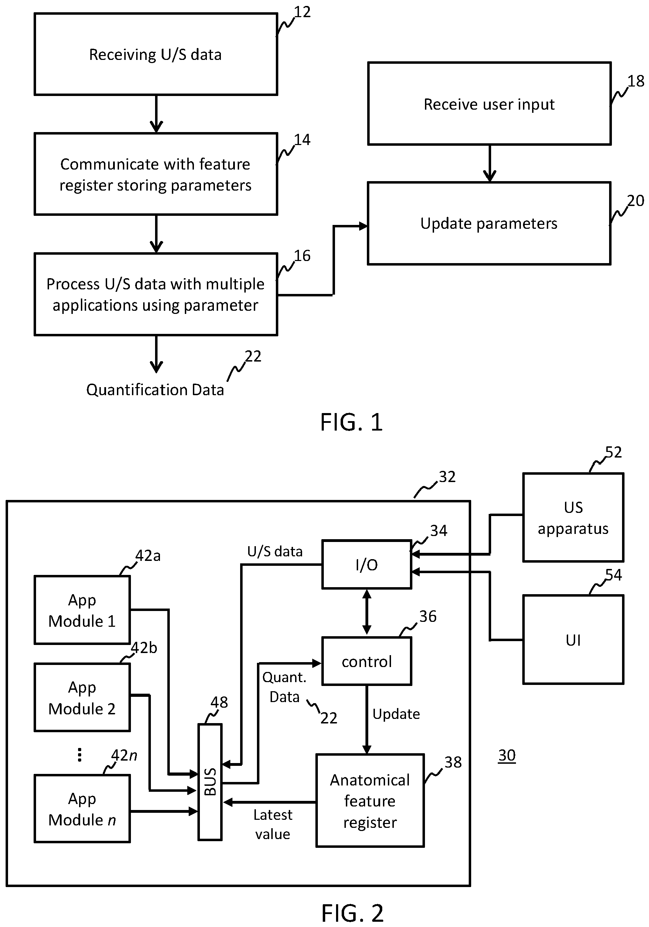

Fig. 1 outlines in block diagram form steps of an example method according to one or more embodiments. The steps will be recited in summary, before being explained further in the form of example embodiments.

The method comprises receiving 12 ultrasound imaging data of an anatomical structure of a patient obtained from an ultrasound acquisition system. The method further comprises communicating 14 with an anatomical feature register which stores one or more parameters associated with one or more anatomical features of the anatomical structure. The method further comprises a plurality of application modules each processing 16 the obtained ultrasound imaging data based on a current value of at least one of the one or more parameters associated with an anatomical feature currently stored in the anatomical feature register to produce output quantification or segmentation data 22.

The method may further comprise receiving 18 input data from a user interface.

The method further comprises dynamically updating 20 the value of at least one of the one or more parameters stored in the anatomical feature register. This may be based on the input data received 18 from the user interface. Additionally or alternatively, it may be based on the output quantification or segmentation data 22 produced by any of the plurality of application modules.

As noted above, the method can also be embodied in hardware form, for example in the form of a processing unit which is configured to carry out a method in accordance with any example or embodiment described in this document, or in accordance with any claim of this application.

To further aid understanding, Fig. 2 presents a schematic representation of an example processing unit 32 configured to execute a method in accordance with one or more embodiments of the invention, and also schematically showing the processing flow in more detail.

The processing unit 32 comprises an input/output 34 for connection in use with an ultrasound data acquisition apparatus 52, for receiving ultrasound imaging data of an anatomical structure of a patient.

The processing unit 32 further comprises an anatomical feature register 38 adapted to store one or more parameters associated with one or more anatomical features of the anatomical structure.

The processing unit 32 further comprises a control module 36 operatively coupled to the anatomical feature register 38. The control module 36 for example comprises one or more processors.

The processing unit 32 further comprises a plurality of application modules 42a, 42b,... 42n operatively coupled to the control module. Each application module is adapted to process ultrasound imaging data received at the input/output 34 based on a current value of at least one of the one or more parameters stored in the anatomical feature register 38 to produce output quantification or segmentation data 22.

The control module 36 may be further be adapted to receive user input data from a user interface 54.

The control module 36 is adapted to dynamically update the value of at least one of the one or more parameters stored in the anatomical feature register 38. This may be based on the user input data provided by the user through the user interface 54. Additionally or alternatively, it may be based on the output quantification or segmentation data 22 produced by any of the plurality of application modules 42a, 42b,...42*n*.

In the schematic illustration of Fig. 2, the processing unit 32 is shown as a singular unitary component within which the various components are located. However, this is not essential. The processing 32 may be a distributed processing unit, with the various components distributed at multiple locations. Furthermore, although the various components are shown as separate hardware modules, this also is not essential. For example, the different application modules 42 may be software modules which are all encoded and executed by a single hardware processing module.

For the purposes of illustration, each of the application modules 42 is shown communicatively coupled with a BUS 48 for facilitating communication between these and each of the input/output 34, the control module 36 and the anatomical feature register 38. The link with the input/output allows the application modules in this example direct access to the incoming ultrasound image data from the ultrasound data acquisition apparatus 52. The link with the control module 36 allows the control module 36 to receive from each of the application modules generated quantification data 22, which may in some cases be used to update the parameter entries in the anatomical feature register 38. The link with the anatomical feature register 38 allows each of the application modules 42 direct access to a latest value of each of the parameters, so that these can be used in the quantifications performed by each module.

An aspect of the invention is the processing unit 32 alone. Another aspect of the invention is a system 30 comprising the processing unit 32 and the ultrasound data acquisition apparatus 52 and/or the user interface device 54.

With regards to the ultrasound data acquisition device 52, this may be an ultrasound scanner. The ultrasound data acquisition device may include a transducer unit (for example an ultrasound probe, or patch). It may further include a local dedicated user interface including a display for displaying ultrasound imagery. The ultrasound data acquisition apparatus 52 may include processing components for processing acquired echo data to generate ultrasound image data.

In operation, the processing and data flow might run, by way of example, as follows.

An ultrasound examination begins, and ultrasound image data begins to be acquired by a user, using the ultrasound data acquisition apparatus 52.

This ultrasound data is received at the input/output 34.

The various application modules 42 begin processing the ultrasound data to derive quantifications of the anatomical structure. Some of the parameters which are stored in the anatomical feature register 38 are quantities which are computed as outputs of one or more of the application modules. These parameters in turn may be inputs used by other of the application modules. Thus, the output quantification or segmentation data of one or more of the application modules may be used to update values of one or more parameters in the anatomical feature register. These parameters may be used by other application modules to perform their own quantifications.

Furthermore, some of the one or more parameters in the feature register 38 might be communicated to the user via the user interface 54 for corroboration or correction of the features. For example a graphical user interface may include fields displaying current values for each of the parameters, and optionally also a source of the current value (e.g. one of the application modules, or initial default values). Furthermore, in some examples, a segmentation generated by a particular application module for the purpose of quantifying dimensions of an anatomical structure could be output on a display to the user interface. The user might utilize a user input element of the user interface to input corrections or amendments to the segmentation. This might then be used by the control module 36 to update relevant dimensional parameters relating to the anatomical structure in the anatomical feature register 38.

In preferred embodiments, the processing unit may further include a register initialization module (not shown) adapted to generate initialization data for at least one of the one or more parameters stored in the anatomical feature register 38 based on the received ultrasound imaging data, and wherein the initialization data comprising an initial value for at least one of the one or more parameters. The control module 36 may be further adapted to trigger the initialization module to generate the initialization data and store the data as an initial value for at least one of the one or more parameters in the anatomical feature register 38.

For example, the initialization data might be generated at a start of the ultrasound examination. For example, the method might further comprises receiving an exam start indicator signal indicative of a start of an ultrasound examination session, for example from the ultrasound acquisition system, and wherein the generating and storing the initialization data is performed responsive to receipt of the exam start indicator signal.

An example implementation of the method according to a particular set of embodiments will now be described by way of illustration of the above-summarized concept of the invention. It will be appreciated that not all features of this particular set of embodiments are essential to the inventive concept, and are described to aid understanding and to provide an example to illustrate the inventive concepts.

This particular set of embodiments relates to an ultrasound system which comprises a number of application modules 42 for computing anatomical and physiological quantities related to the heart.

Thus, in this set of embodiments, the anatomical structure is a cardiac structure. By way of example, the one or more parameters associated with an anatomical feature stored in the register might include one or more of the following: a heart size; a left ventricle (LV) size; a left atrium size; a right ventricle (RV) size; a right atrium size; LV length; RV length; regional wall thicknesses; LV diameter. In some embodiments, the one or more parameters may further include one or more segmentation masks (or representations thereof) for one or more structures, such as for the LV or RV. A segmentation mask can be stored as contour which consists of multiple x/y points. These points could be treated and stored in the register as anatomical parameters (scalars) that can be put into a vector, e.g. one might have 20 pairs of x/y image coordinates describing an LV contour. The reference to 'size' above, e.g. of the heart or LV or RV, could be for instance an area parameter (e.g. across a 2D plane) or volume parameter (e.g. estimated from a 2D cross-section or derived from 3D data).

There may furthermore be parameter values recorded for each parameter at both end systole and end diastole. The parameters may further include dynamic parameters such as for instance: ejection faction, cardiac output, or strain parameters.

The complete set of parameters might be stored as a vector, known as a heart size or heart shape vector, with each element of this vector corresponding to one of the parameters.

The heart size or heart shape vector is saved in the register and available to all of the application modules. The heart size or heart shape vector can be updated throughout the exam.

For example, initial values of the different parameters contained in the heart size/shape vector might be set, for instance based on prior patient scan data, or using a separate algorithm (to be described in more detail later).

Following this, more refined values for at least a subset of the parameters might be obtainable from quantifications performed by one or more of the application modules 42. For example, at least one of the application modules may be adapted to output a value for heart size or left ventricle size, and this could be used to update these parameter values. Other of the applications might use the heart size and/or shape vector to compute other quantifications such as cardiac output. Certain size or dimensional parameters may be used as bases to compute other size or dimensional parameters.

Additionally, based on the current values of the parameter in the register, acquisition parameters of the ultrasound data acquisition apparatus might be determined and communicated to the apparatus.

By way of example, in one system, there are a plurality of application modules which each receive as input a 3D ultrasound image and produce as output a segmentation map of the heart (in the form of a mesh). Each of these example application modules starts its process of generating the segmentation map with an initial heart shape. This heart shape can be scaled by an additional input such as the heart size. This scaling then helps each of the application modules to converge to a better segmentation or to prevent failure. Besides heart size, other scaling parameters could be used, such as LV size or any of the other parameters mentioned in this disclosure.

Thus, as a principle of more general application for any embodiment of this invention, for at least a subset of the plurality of application modules, the output quantification or segmentation data may include a segmentation of at least one anatomical feature or structure, and wherein the application module is adapted to generate the segmentation based on an initial shape template, and wherein the initial shape template is scaled according to a scaling parameter, and wherein the scaling parameter is, or is computed based on, at least one of the one or more parameters stored in the anatomical feature register. The resulting segmentation mesh or mask can be used for a number of different purposes. It may for example be used to compute one or more quantifications, to therefore generate quantification data. For example, the one or more quantifications may include measurements such as ejection fraction, heart size, LV size, etc. It can also be used derive 2D planar images from the 3D input image. Thus, in some examples, the ultimate output from the application module may be quantification information, and wherein a segmentation is derived as a interim step, based on one or more of the parameters in the register as an input.

Additionally or alternatively, in some examples, the plurality of application modules may include one or more modules configured to quantify strain information, such as global longitudinal strain. For example, at least one module may be configured to receive as input a 2D ultrasound image and to generate as output the strain information. Here, the one or more parameters in the feature register may be used to generate an initial estimate of the quantified strain information which is then refined for example iteratively. The output is a strain measurement.

In some examples, one or more of the application modules may be adapted to use one or more of the parameters stored in the anatomical feature register for a quality control check. For example, a quality control check might be applied to a candidate value of a particular anatomical quantification based on reference to one or more parameter values in the register, and, if the candidate value passes the quality control check then the candidate value is output as the output quantification data from the application module.

For example, an application module might be configured to compute a heart size or LV size based on an input ultrasound image. A value of the parameters in the feature register may not be needed for this purpose. For example, an initial estimate of the heart size may not be needed. However, after the application module computes a candidate value for the heart size, it could be compared with an existing value of the heart size in the feature register to check if it is within a pre-defined range of the existing value in the feature register. If outside of the pre-defined range, then the quality control check fails, and the candidate value may be refined or discarded for example.

Additionally or alternatively, in some embodiments, two or more of the application modules may be configured to compute a same one of the parameters which are stored in the feature register, and wherein the method comprises performing a comparison of the multiple generated values of said parameter to one another to check consistency. If consistency meets a pre-defined consistency threshold, e.g. the values of the parameters are within a pre-defined range of one another, then the register can be updated based on the generated parameter values, e.g. selecting one of the parameters values based on a pre-defined criterion or computing an average value. If the consistency does not meet the pre-defined consistency threshold, then the method may reject the generated parameter values, and may for example wait for the values to be re-computed by the two or more application modules.

As mentioned above, in some embodiments, an initial value of at least one parameter can be derived, for example using an initialization module. For example, a first estimate of the heart size, or the left ventricle size can be derived by applying a dedicated initialization algorithm as soon as an image covering the first apical chamber has been acquired. By way of example, a deep learning network could be applied to acquired image data to acquire a first value of the heart size or left ventricle size. As a more general principle, it is proposed that initialization data for at least one of the one or more parameters can be derived by applying an artificial intelligence algorithm to received ultrasound imaging data. The artificial intelligence algorithm may be a convolutional neural network in advantageous examples.

As also mentioned above, additionally or alternatively, initialization data for at least one of the one or more parameters may be derived based on accessing a data record in a datastore associated with the patient who is being examined. In some embodiments for example, historical image data associated with the patient can be accessed, and a quantification algorithm applied to this historical image data to derive an initial value for at least one of the one or more parameters. As a simpler example, the datastore may record a most recent value of the parameter previously computed, and this could be used as the initialization data for the parameter. For example, in the case of longitudinal scanning, the previous heart size can be determined from a record in a PACS system and used as the initial estimate.

The initial value for the one or more parameters may be utilized by one or more of the plurality of application modules for initial quantifications.

As already discussed, it is proposed that functionality be provided by means of which a user can update or correct one or more of the parameters in the anatomical feature register. For example, each parameter might be assigned an initial value at a start of the examination (in accordance with initialization data), where this might be a default value, or might be a value obtained from a data record associated with the patient, or might be a value computed by a dedicated algorithm or application module.

The user interface device 54 may display a graphical user interface window on its display unit, and this may include fields which indicate a current value of each of the plurality of parameters associated with anatomical features of the anatomical structure. For example, there may be a field that indicates the current estimate of the heart size, LV size, or any of the other parameters mentioned previously. Furthermore, the graphical user interface may further indicate for each parameter the source of the parameter value, e.g. initialization data (e.g. a deep learning neural network), historical patient data, a specified one of the application modules, or user-input data. The user interface may be configured to permit a user to input amended values for at least a subset of these values. In other words, the user can override the information. Particularly at a beginning of the exam, before the application modules 42 have generated quantification data, this may be valuable. The various parameters are stored in the register in the background.

In state of the art ultrasound systems, all application modules for performing quantifications operate independently of one another. This means that if more than one application module utilizes a same input parameter related to the heart size, then this parameter has to be updated multiple times, once for each application. Embodiments of the present invention provide improved sharing of parameters, among other benefits.

These initial values might remain in place in the register for use by each of the application modules 42 to which they are relevant unless and until updated.

Additionally or alternatively, a user can input updates or corrections of one or more of the parameters, e.g. a manual correction to a segmentation, via the user interface 54.

It is proposed in accordance with some embodiments that once a user has updated a given parameter, it should be locked for editing or updating for the remainder of the given examination. For example, this could be implemented by means of an update counter entry included in the anatomical feature register 38 associated with at least one of the one or more parameters. It is proposed that, concurrently with any updating of the parameter value in the anatomical feature register based on user input data, the update counter entry be incremented, and wherein any update of the parameter in the anatomical feature register is blocked responsive to a current value of the update counter register exceeding a threshold. This threshold could be set at zero, so that once a user has updated or corrected a parameter once in a given examination, it remains locked for the rest of the examination.

A reset between examination sessions could be implemented by means of a reset indicator signal generated at an end or at a beginning of any ultrasound examination session, and wherein the method comprises resetting the update counter entry to zero responsive to the reset indicator signal.

As noted above, in some embodiments, initialization data is generated to provide an initial value for one or more of the parameters stored in the anatomical feature register. In some embodiments, it is proposed to use an artificial intelligence algorithm, applicable to input ultrasound image data, to compute the initialization data.

The inventors have developed a new convolutional neural network for this purpose.

The generated model is a deep learning model for estimating heart size. It can be designed as convolutional neural network (CNN) that takes as input a 2D, 3D, 2D+t, or 3D+t ultrasound image data set, and generates as output a heart size estimate. The convolutional neural network can be configured in an end-to-end manner such that the heart size parameters are directly regressed as continuous parameters.

A convolutional neural network (CNN) in accordance with this description was implemented, trained, and tested by the inventors. The CNN was trained to predict end-diastolic (ED) and end-systolic (ES) Left-ventricular (LV) volume based on an input 2D+t ultrasound image set. A 2D+t image data set comprises a series of 2D image frames, each corresponding to a different time point.

The CNN uses 3D convolutions and consists of seven convolutional layers, a global average pooling layer, and an output layer with two outputs. There are three resolution stages. Downsampling between stages is performed with a stride of two inside the convolutions. The model was trained using a training dataset of 25000 ultrasound DICOM (2D+t) image data sets.

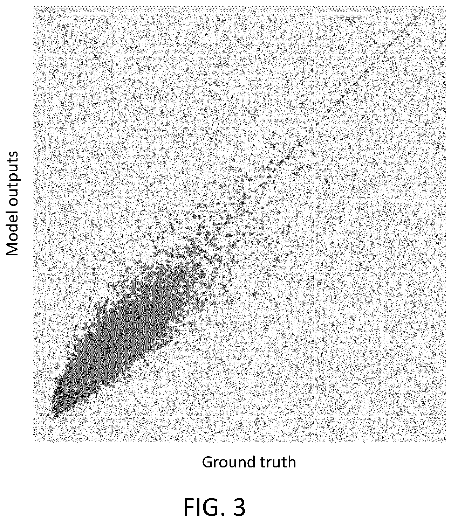

The resulting performance of the CNN model is illustrated in Fig. 3. This shows the correlation between the prediction values for the heart size (y-axis) and the ground truth values (x-axis). Quantitatively, the CNN achieves a mean absolute error of 18.60 ml for ED volume and 11.44 ml for ES volume over a large range of volumes (see Figure 2). The Pearson's correlation coefficient is 0.864 for ED volume and 0.899 for ES volume. As an estimate for the rough heart size this performance is adequate.

Where the invention is provided embodied in hardware, there are different options as to how this hardware can be configured. For example, a physically separate processing unit could be provided, e.g. as illustrated in Fig. 2. In other embodiments, some or all of the functionality ascribed above to a single processing unit could be distributed between multiple processing devices. In some embodiments the functionality of the processing unit 32 could be integrated in a processing unit of the ultrasound data acquisition apparatus 52. In some embodiments, the processing unit may be a cloud-based processing unit, and wherein communication with the ultrasound apparatus 52 and the user interface 54 is facilitated via an Internet link.

As mentioned previously, the invention can be embodied in software form. Thus another aspect of the invention is a computer program product comprising code means configured, when run on a processor, to cause the processor to perform a method in accordance with example or embodiment of the invention described in this document, or in accordance with any claim of this patent application.

Some embodiments of the invention described above employ a processing unit. The processing unit may in general comprise a single processor or a plurality of processors. It may be located in a single containing device, structure or unit, or it may be distributed between a plurality of different devices, structures or units. Reference therefore to the processing unit being adapted or configured to perform a particular step or task may correspond to that step or task being performed by any one or more of a plurality of processing components, either alone or in combination. The skilled person will understand how such a distributed processing arrangement can be implemented. The processing unit may include a communication module or input/output for receiving data and outputting data to further components.

The one or more processors of the processing unit can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A computer-implemented method comprising:
receiving (12) ultrasound imaging data of an anatomical structure of a patient obtained from an ultrasound acquisition system;
communicating (14) with an anatomical feature register which stores one or more parameters associated with an anatomical feature of the anatomical structure;
a plurality of application modules each processing (16) the obtained ultrasound imaging data based on a current value of at least one of the one or more parameters associated with an anatomical feature currently stored in the anatomical feature register to produce output quantification or segmentation data (22); and
receiving (18) input data from a user interface;
wherein the method further comprises:
dynamically updating (20) the value of at least one of the one or more parameters stored in the anatomical feature register based on the input data received from the user interface and/or the output quantification data produced by any of the plurality of application modules.

2. The method of claim 1, further comprising
generating initialization data for at least one of the one or more parameters of the anatomical feature based on the received ultrasound imaging data, and
storing the initialization data in the anatomical feature register as an initial value for the at least one of the one or more parameters.

3. The method of claim 2, wherein
the method further comprises receiving an exam start indicator signal indicative of a start of an ultrasound examination session, for example from the ultrasound acquisition system, and
wherein the generating and storing the initialization data is performed responsive to receipt of the exam start indicator signal.

4. The method of claim 2 or 3, wherein generating the initialization data for the at least one of the one or more parameters comprises applying an artificial intelligence algorithm to the received ultrasound imaging data.

5. The method of claim 4, wherein the artificial intelligence algorithm is a convolutional neural network.

6. The method of any of claims 1-5, wherein the anatomical structure is a cardiac structure, and wherein the one or more parameters include one or more of: a heart size, a left ventricle size, a left atrium size, a right ventricle size, a right atrium size at end diastole, ejection faction and/or a left ventricle length.

7. The method of any of claims 1-6, wherein the received ultrasound imaging data comprises: 2D ultrasound imaging data for one or a series of time frames, or 3D ultrasound imaging data for one or a series of time frames.

8. The method of any of claims 1-7, wherein the anatomical feature register includes an update counter entry associated with at least one of the one or more parameters, and wherein concurrently with any updating of the parameter value in the anatomical feature register based on the user input data, the update counter entry is incremented, and wherein any update of the parameter in the anatomical feature register is blocked responsive to a current value of the update counter register exceeding a threshold.

9. The method of claim 8, wherein the threshold is zero.

10. The method of claim 8 or 9, wherein a reset indicator signal is generated at an end or at a beginning of any ultrasound examination session, and wherein the method comprises resetting the update counter entry to zero responsive to the reset indicator signal.

11. A computer program product comprising code means configured, when executed by a processor, to perform a method in accordance with any of claims 1-10.

12. A processing unit (32), comprising:
an input/output (34) for connection in use with an ultrasound data acquisition apparatus, for receiving ultrasound imaging data of an anatomical structure of a patient;
an anatomical feature register (38) adapted to store one or more parameters associated with an anatomical feature of the anatomical structure;
a control module (36) operatively coupled to the anatomical feature register;
a plurality of application modules (42a, 42b,... 42n) operatively coupled to the control module, each application module being adapted to process ultrasound imaging data received at the input/output based on a current value of at least one of the one or more parameters stored in the anatomical feature register to produce output quantification or segmentation data (22); and
wherein the control module is adapted to:
receive user input data from a user interface (54); and
dynamically update the value of at least one of the one or more parameters stored in the anatomical feature register based on the user input data provided by the user through the user interface and/or the output quantification or segmentation data produced by any of the plurality of application modules.

13. The processing unit of claim 12, further comprising:
a register initialization module adapted to generate initialization data for at least one of the one or more parameters based on the received ultrasound imaging data, the initialization data comprising an initial value for at least one of the one or more parameters, and
wherein the control module is further adapted to trigger the initialization module to generate the initialization data and store the data as an initial value for at least one of the one or more parameters in the anatomical feature register.

14. The processing unit of claim 12 or 13, wherein the anatomical structure is a cardiac structure, and wherein the one or more parameters include one or more of: a heart size, a left ventricle size, a left atrium size, a right ventricle size, a right atrium size at end diastole, ejection faction and/or a left ventricle length.

15. A system (30) comprising:
the processing unit (32) of any of claims 12-14; and
an ultrasound data acquisition apparatus (52) operably coupled with the input/output of the processing unit, and
optionally further comprising a user interface (54) for obtaining a user input.
